(19) **Europäisches Patentamt / European Patent Office / Office européen des brevets**

(11) **EP 2 287 701 A1**

(12) **EUROPEAN PATENT APPLICATION**
published in accordance with Art. 153(4) EPC

(43) Date of publication:
**23.02.2011 Bulletin 2011/08**

(21) Application number: **08706570.2**

(22) Date of filing: **28.01.2008**

(51) Int Cl.:
*G05D 27/00* (2006.01)    *G05D 23/00* (2006.01)
*G05D 22/00* (2006.01)    *G01N 33/46* (2006.01)

(86) International application number:
**PCT/CN2008/070193**

(87) International publication number:
**WO 2009/094860 (06.08.2009 Gazette 2009/32)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Research Institute Of Wood Industry Chinese Academy Of Forestry Beijing 100091 (CN)**

(72) Inventors:
• **ZHOU, Yucheng**
  **Beijing 100091 (CN)**
• **CHENG, Fang**
  **Beijing 100091 (CN)**
• **AN, Yuan**
  **Beijing 100091 (CN)**
• **ZHANG, Xingmei**
  **Beijing 100091 (CN)**

(74) Representative: **Langenhuijsen, Bastiaan Wilhelmus Herman Patentwerk B.V. P.O. Box 1514 5200 BN 's-Hertogenbosch (NL)**

(54) **A DYNAMIC AND TRACING SYSTEM FOR TEST ENVIRONMENT THAT CONTAINS THE AMOUNT OF FORMALDEHYDE AND VOC RELEASED FROM ARTIFICAL BOARD**

(57) The present invention discloses a dynamic tracing control system for the testing environment of formaldehyde and volatile organic compound emissions from wood-based panels, comprising a computer, a programmable logic controller PLC, a test chamber, a temperature control subsystem and a humidity control subsystem. The temperature control subsystem is used to heat the testing environment of the test chamber. The humidity control subsystem is used to supply the test chamber with air of defined pressure, temperature and humidity. The control program executed by the PLC takes into account such interference factors as outside environment, condition, and atmospheric pressure. After the temperature and humidity signals of the test chamber are collected, a nonlinear mathematical model is established. Decoupling of this mathematical model uses diffeomorphism transformation and dynamic compensator, thus accurately calculating the outputs of the system. This method greatly shortens the time required to achieve the testing conditions, and improves the test accuracy by 7% and saves energy by 10% compared to the control system in the background art.

Figure 1

## Description

## Technical Field

The present invention relates to a control system for the testing environment of formaldehyde and volatile organic compound (VOC) emissions from wood-based panels, and more particularly to a dynamic tracing control system of temperature and humidity in the testing environment of formaldehyde and VOC emissions. The system is used to determine the content of harmful volatile gases in the environment, forest products, building materials and chemical products.

## Background Art

Forest products (such as furniture, plywood, MDF, fiberboard), building materials and chemical products are essential to the daily life of people. Most of these products need to use formaldehyde and volatile organic compounds (VOC) for production. Herein, VOC is the abbreviation of volatile organic compounds (VOCs). The present invention adopts the World Health Organization's (WHO, 1989) definition of the total volatile organic compounds (TVOC), i.e., the general terms of volatile organic compounds with a melting point lower than room temperature and a boiling point between 50˚ and 260˚. As the boiling point of formaldehyde is not between 50˚ and 260˚, formaldehyde is not included therein. Formaldehyde is one of the main raw materials for the synthesis of urea formaldehyde resin adhesive, and is a volatile substance. The wood-based panels made of formaldehyde and products thereof are the main indoor air pollutant. Formaldehyde in them can be released into the air, and exposure to a high concentration of indoor formaldehyde can lead to human cancers or death. At present, there is no substitute for formaldehyde. The volatile organic compounds released from the wood-based panels are also a threat to human health, and can cause impaired lung functions, central nervous system disorder, and reduce the body's ability to fight diseases. Moreover, in normal interior trims, the releasing of formaldehyde from wood-based panels can last 3 to 15 years. In the wood-based panel industry both at home and abroad, more than 90% of the wood-based panels use adhesives containing formaldehyde. However, to date there are no other synthesized monomers that are cheaper and have better bonding performance than formaldehyde. In view of this, the mandatory national standard of China, *Formaldehyde Emission Limit of Wood-Based Panels and Products Thereof as Indoor Trims and Finish Materials,* has been implemented beginning January 1, 2002 with a transitional period of six months. The national mandatory standard stipulates that any products which do not meet the standard must not be sold within the territory of China beginning July 1, 2002. China has become the number one country in wood-based panel production. But the emissions of formaldehyde and VOC from the wood-based panels do not meet the requirements of the mandatory national standard, which causes deterioration of people's living and work environment, threatens human health, and limits the exports. Therefore, it is very important to test the harmful gases in the forestry products and building finish materials.

To meet the requirements of the Chinese national standard, it is essential to test the harmful gases in the products to ensure safe, stable, efficient, high-quality, and low-consumption production. Formaldehyde and VOCs are volatile substances. Today, there are four methods for testing formaldehyde in the world, i.e., perforation extraction method, desiccator method, climate chamber method and the new gasometry method. Of these four methods, the climate chamber method is internationally believed to produce true and reliable results, and the climate chamber method is used in many countries including China to determine the formaldehyde and VOC emissions from the wood-based panels and products thereof. To use the climate chamber method to determine the formaldehyde emission, the tested sample is placed in the test chamber of defined temperature, humidity and air circulation rate. After a certain length of time, the gases in the test chamber are extracted for physical or chemical analysis. Finally the formaldehyde emission from the tested sample is determined. This method has very demanding requirements on the test chamber. In particular, the test chamber should have a climatic environment that has constant and precisely-controlled internal temperature and humidity. That is to say, the test chamber is time dependent and a certain amount of gas of certain temperature and humidity is fed to the chamber from the outside in a time unit. At the same time, the chamber outlets a certain amount of gases. Throughout the testing process, no condensation in the chamber is allowed because formaldehyde or part of VOC is water soluble. The test result can be affected if there is condensation. This requires that the chamber internal temperature and humidity should be set within the allowed tolerance, and the reliability and accuracy of the test results depend on the accuracy of temperature and humidity inside the chamber.

Prior to the present invention, the tabulation method invented by German scientists is used by all to control the internal temperature and humidity of the testing environment of formaldehyde and volatile organic compound emissions. This tabulation method makes the vapor pressure, dry bulb temperature, wet bulb temperature and air circulation speed inside the testing environment relatively fixed. A set of tables are produced by experimental methods. During the test, the temperature and humidity values used in the test are looked up in the tables, and then the testing environment is controlled to the required temperature and humidity by controlling the dew point temperature.

This method of controlling the temperature and humidity of the testing environment has a number of disadvantages both in the test results and in the adjustment method. For example, it is easily affected by external interference, which leads to very poor reliability and inability of the testing environment to meet the requirements. Therefore, the accuracy of controlling the testing environment of formaldehyde and volatile organic compound emissions is a problem which all countries in the world have been trying to address.

The inventor of the present application has filed an invention patent ZL03105211.8 with China entitled "A robust tracing control method of the gas temperature and humidity inside an artificial climate chamber" and a utility model ZL03204471.2 entitled "A gas generating device for the artificial climate chamber with a robust tracing control method". In these two patents of the background art, a gas generating device for the artificial climate chamber used to determine the atmospheric gases or volatile harmful gases in products and the robust tracing control method for controlling said gas generating device are disclosed. Said gas generating device includes an artificial climate chamber, a constant-temperature water tank heating device used for heating the testing environment inside the artificial climate chamber, a constant-temperature dew point gas inlet device used for providing air of certain pressure, temperature and humidity to the artificial climate chamber and a control system comprising a programmable logic controller (PLC). The PLC uses the robust tracing control method to control the testing environment inside the artificial climate chamber to the required temperature and humidity. The robust tracing control method using PLC is real-time acquisition of the temperature and humidity inside the climate chamber, comparing the measured values with the preset values and taking the difference between the actual values and the preset values for output adjustment. The existing problem is that the control system requires a longer time and higher energy consumption to reach the preset values and cannot meet the requirements of high efficiency and high precision. In addition, as most of the hardware used in the gas generating device adopts manual regulating valves for manual adjustment and the PLC does not control these regulating valves, this results in the problems of long testing time, low testing accuracy and high energy consumption.

**Disclosure of the Invention:**

In view of these problems, the inventor of the present application makes improvements from the patents mentioned in the background art, and proposes a dynamic tracing control system for the testing environment of formaldehyde and volatile organic compound emissions from wood-based panels. The method of the invention has the benefits of quick testing, high precision, and low energy consumption. The hardware structure has been improved, including: 1) an electric air regulating valve is added following the air filter; 2) an electric moisture regulating valve is used in the place of the manual moisture regulating valve to control the amount of moisture supplied into the test chamber; 3) the original scheme in which part of the circulating air in the test chamber flows through the heat exchanger is replaced by one in which all of the circulating air flows through the heat exchanger, so as to shorten the time required for the testing environment in the chamber to reach the required test conditions by one third, and to reduce the energy use by 3-5%.

More importantly, the dynamic tracing control method of the hardware according to the present invention is fundamentally different from the robust tracing control method in the background art. The dynamic tracing control method according to the present invention is characterized in that a nonlinear mathematical model is established after temperature and humidity signals of the test chamber are collected. By the diffeomorphism transformation and the dynamic compensator, this model is decoupled, and thereby the outputs of the system are calculated accurately. This method significantly shortens the time needed to attain the required test conditions, improves test accuracy and saves energy. Compared with the control method in the two patents of the inventor, this method increased the control precision by 7% and saved energy by 10%.

The present invention solves the problems of the result of the tabulation method with poor stability and bad reliability, which is used by most countries in the world to control the testing environment of formaldehyde and VOC emissions from wood-based panels. Further, it solves the problem of high-precision control of the testing environment for formaldehyde and VOC emissions, which has long been a worldwide subject.

The inventor considers the testing environment of formaldehyde and VOC emissions from wood-based panels as a complex system. First, a systemic dynamics mathematical model is established and proposed, which is in the nonlinear singular form. By diffeomorphism transformation, the system is transformed into the controllable and observable form. Then by discretizing the system, the discrete-time system nonlinear singular control dynamic model is obtained. The dynamic tracing control of the system is finally realized by the disturbance decoupling of the complex discrete-time and nonlinear singular control system.

Based on the above principle, the technical scheme is a dynamic tracing control system for the testing environment of formaldehyde and VOC emissions from wood-based panels, comprising a computer, a PLC, a test chamber, a temperature control subsystem and a humidity control subsystem; wherein the computer is connected to the PLC, and the temperature control subsystem and the humidity control subsystem are connected to the test chamber respectively; the temperature control subsystem is used for heating the testing environment in the test chamber, and said humidity control subsystem supplies the test chamber with the air with a certain pressure, temperature and humidity;

the temperature and humidity sensor is set in the test chamber, and the PLC is connected to said temperature and humidity sensor, the temperature control subsystem and the humidity control subsystem respectively, and the PLC controls said temperature control subsystem and humidity control subsystem according to the signals collected by said temperature and humidity sensor, so as to control the testing environment inside the test chamber to the required temperature and humidity;

the control system is characterized in that, said control program of the PLC is made on the premise of considering interference factors, such as external environment, condition and atmospheric pressure, and the temperature and humidity are master variables represented by $x_n(t)$; the uncertainty in the climatic chamber, such as dry bulb temperature, wet bulb temperature and vapor pressure, is regarded as the internal disturbance represented by $Q_n(t)$; the uncertainty of external environment, such as temperature, humidity and atmospheric pressure, is regarded as the external disturbance represented by $q_n(t)$; $x_n(t)$ is input, $y_n(t)$ is output, and $z(t)$ is feedback; according to the settings mentioned above, a discretized system dynamics model system $\sum_1$ for the testing environment in test chamber is established:

$$x(k+1) = f(x(k), z(k), u(k), w(k))$$

$$g(x(k), z(k), u(k)) = 0$$

$$y(k) = h(x(k))$$

where the state variable is $x(k) \in X^0 \subset R^n$, the bounding variable is $z(k) \in Z \subset R^l$ the control variable is $u(k) \in U \subset R^m$, the disturbing variable is $w(k) \in W \subset R^m$, the output variable is $y(k) \in Y \subset R^p$, R is the real number, k, l, m, n and p are integers, $p \leq m$ ; $X^0$, Z, U, W are respectively open sets of space $R^n, R^l, R^m R^r$, Y is an open neighborhood in $R^m$, arbitrary order derivatives of function f, g and h exist and are continuous; the following calculations are executed for said system $\sum_1$:

A) define:

$$\rho = rank \frac{\partial}{\partial(\sigma x, z)} F^1(\sigma x, x, z, u, w) =$$

$$rank \frac{\partial}{\partial(\sigma x, z)} \left[ \begin{array}{c} f(\sigma x, x, z, u, w) \\ g(x, u, z) \end{array} \right]_{(x_e, u_e, w, z_e)}$$

where $F^1 = (f^T, g^T)^T, \sigma^\alpha x(k) = x(k+\alpha)$, $\sigma$ is differential operator, $\alpha$ is differential order, the equilibrium point of system $\sum_1$ is $(x_e, z_e, u_e, w_e)$, and it satisfies $f(x_e, x_e, z_e, u_e, w_e) = 0$
and
$g(x_e, z_e, u_e) = 0$
B) choose a dynamic compensator

$$T_1(k) = u(k)$$
$$T_2(k) = u(k+1)$$
$$\vdots$$
$$T_{\alpha-1}(k) = u(k+\alpha-1)$$
$$u(k+\alpha) = v(k)$$

C) by using the calculation mentioned above, the discrete-time singular system $\sum_1$ is transformed into the following form, and it is denoted by

$$x(t+1) = f(x(x), u(t), w(t)).x(0) = x_0$$
$$\sum : y(t) = h(x(t))$$

where the state variable is $x(t) \in X^0 \subset R^n$, the control variable is $u(t) \in U \subset R^m$, the disturbing variable is $w(t) \in W \subset R^r$, the output variable is $y(t) \in Y \subset R^p$; t is the time variable, $X^0, U, W$ are respectively open sets of space $R^n, R^m, R^r$; Y is an open neighborhood in $R^P$, mappings $f : X \times U \times W \rightarrow X, h : X \rightarrow Y$ are analytical;

D) the mapping $f(*)$ defines an immersion, i.e., $rank_k \dfrac{\partial f}{\partial(x,u)} = n$

E) by using the inversion algorithm again for the system $\Sigma$, choose the feedback control as follows:

$$u^1(t) = \varphi(x(t), \tilde{v}_i(t+r), v^2(t))$$

$$u^2(t) = v^2(t)$$

where $1 \leq i \leq \alpha, 0 \leq r \leq \alpha - i$
$u^1(t)$
and $u^2(t)$ are outputs of system $\Sigma$, which means the system can achieve the state set by the user by controlling this variable;

F) by using the inversion algorithm again, the following expression is derived $\tilde{y}_k^u(t+k) = \tilde{v}_k(t), t \geq 0$ and for $k \geq 0$, the expression is

$$d\hat{y}_k^u(k) - \sum_{i=1}^{k} \sum_{j=i}^{k} \overline{e}_k^{ij} d\tilde{y}_i(j) \in X$$

thereby the temperature and humidity required by the system $\Sigma_1$, i.e., output variable $\hat{y}_k^u(k)$, is obtained. Moreover, said temperature control subsystem further comprises a constant temperature water tank, a water pump and a heat exchanger which is set in the test chamber; said PLC can control the heaters in the constant temperature water tank according to the above-mentioned dynamic tracing control program, thereby controlling the temperature of the water tank, and it can also control the water supply of the heat exchanger by controlling the water pump operation.

Furthermore, said humidity control subsystem further comprises an air filter, an air regulating valve, an air pump, a humidity generator, a humidity regulating valve and a humidifier, which are connected with moisture pipes; in addition, said humidity control subsystem also comprises a dew point water tank and a magnetic pump, which are connected with water pipes; said test chamber comprises a humidifier connected to said humidity regulating valve through moisture pipes, a heat exchanger connected to said water pump through hot water pipes and fans connected to the PLC; by the control bus, the PLC controls the constant temperature water tank, dew point water tank, air regulating valve, magnetic pump, humidity regulating valve, air pump, refrigerator, water pump and fans.

The advantage of the present application is that, all the disturbances of the abstracted system dynamics model are regarded as a variable family. From this point of view, the control of the disturbed system is considered to shorten the time for the testing environment to reach the required test conditions by at least one third compared to the existing control methods used at home and abroad; the system dynamic model is established specifically for the actual system, and the intelligent diagnosis of the system is realized by the inversion algorithm, so as to output the system optimal control variables, improve the control precision and save energy significantly.

Description of the Drawings

Figure 1 is the structure diagram of a dynamic tracing control system for the testing environment of formaldehyde and VOC emissions from wood-based panels;

Figure 2 is the modeling theoretical diagram of the PLC according to the present invention;

Figure 3 is the cover page of the present invention displayed on the computer;

Figure 4 shows the parameters setting interface of the present invention displayed on the computer;

Figure 5 is the "user management" interface of the present invention displayed on the computer;

Figure 6 is the main interface of the present invention displayed on the computer;

Figure 7 is the interface displayed on the computer for confirming the set parameters of the present invention;

Figure 8 is the system starting interface of the invention displayed on the computer;

Figure 9 is the process curve interface of the climate chamber in the present invention attaining the testing requirements.

**Particular Embodiments**

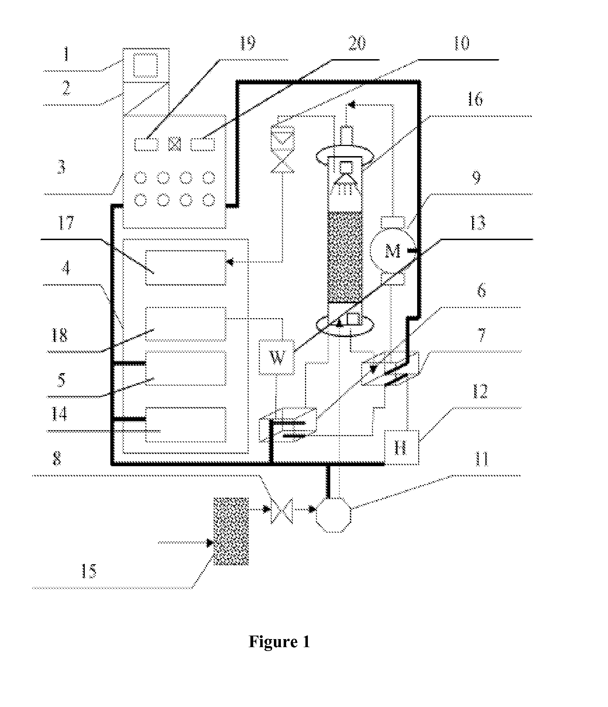

The preferred embodiments are described below with reference to the accompanying drawings. First, the hardware structure of the present application is described with reference to Figure 1. The hardware structure of the invention comprises a computer 1, a PLC 3, a humidity control subsystem, a temperature control subsystem and a test chamber. The connection between the computer 1 and the programmable logic controller 3 uses a RS485-232 converter 2; according to the temperature and humidity signals acquired from the sensor 5 in the test chamber 4, the PLC controls the switches of the constant temperature water tank 6, the dew point water tank 7, the air regulating valve 8, the oilless, magnetic pump 9, the moisture regulating valve 10, the air pump 11, the refrigerator 12, the water pump 13 and the fan 14 via the control bus. The humidity control subsystem comprises an air filter 15, an air regulating valve 8, an air pump 11, a humidity generator 16, a moisture regulating valve 10 and a humidifier 17, which is connected by the moisture pipes; in addition, the humidity control subsystem also comprises a dew point water tank 7 and a magnetic pump 9 which are connected by the water pipes; the temperature control subsystem comprises a constant temperature water tank 6, a water pump 13 and a heat exchanger 18, which are connected by water heating pipes. In addition, the heater and refrigerator in the constant temperature water tank are connected to the PLC via the control bus. The test chamber 4 has a humidifier 17 which is connected to the moisture regulating valve 10 by the moisture pipes, a heat exchanger 18 which is connected to the water pump 13 by the heating water pipes and a sensor 5 which is connected to the PLC through the control bus, and a fan 14.

In the application, a temperature and humidity sensor 5, the humidity sensor being a high molecular humidity sensitive capacitor and the temperature sensor being Pt100, are set in the climate chamber 4, which can respectively detect the temperature and humidity standard signals. The signals are sent to the temperature display instrument 19 and the humidity display instrument 20 (model DM604) with transforming output through the shielded cable. The temperature display instrument 19 and humidity display instrument 20 transmit the data to the temperature and humidity conversion module SIEMANS EM231 which transmits the signals to the PLC. After the PLC processes the signals, the temperature and humidity sampling values in the test chamber are obtained. When the user sets the temperature and humidity values required in the climate chamber on the operating computer and transmits these values to the PLC through the configuration program, the PLC implements the appropriate dynamic tracing control program according to the actual temperature and humidity sample values inside the test chamber, and controls the temperature of the constant temperature water tank 6 and the dew point water tank 7 in real time until the temperature and humidity inside the test chamber reach the preset requirements. Meanwhile the PLC is also responsible for controlling the normal operation of the air pump 11, the refrigerator 12, the water pump 13 and the magnetic pump 9. At the same time, the PLC downloads and receives the computer's control signals, runs the required judging, dynamic tracing and programs.

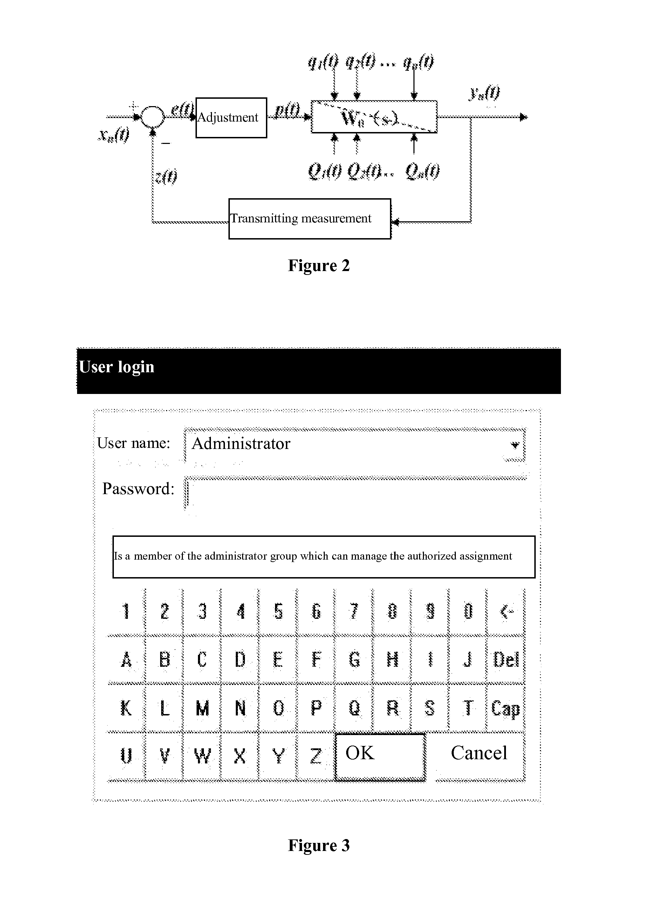

The modeling theoretical diagram of the invention is shown in Figure 2. The model is abstracted from the control system by taking into account many factors such as external environment, conditions, atmosphere pressure and the interference. The temperature and humidity are master variables represented by $x_n(t)$ in the model; the uncertainty in the climatic chamber, such as dry bulb temperature, wet bulb temperature and vapor pressure, is regarded as the internal disturbance represented by $Q_n(t)$ ; the uncertainty of external environment, such as temperature, humidity and atmospheric pressure, is regarded as the external disturbance represented by $q_n(t)$.

$x_n(t)$ is input, $y_n(t)$ is output, and $z(t)$ is feedback.

First, the system is abstracted, and a continuous-time system dynamic model is established. Then the discretized system $\sum_1$ of said system is obtained by discretization, wherein, $z(k)$ includes the external disturbances $q_n(t)$ and internal disturbances $Q_n(t)$, and $u(k)$ includes feedback. The system $\sum_1$ can be represented as follows: $x(k+1) = f(x(k), z(k),u(k),w(k))$

(1)

$$g(x(k), z(k), u(k)) = 0 \qquad (2)$$

$$y(k) = h(x(k)) \qquad (3)$$

where the state variable is $x(k) \in X^o \subset R^n$, the bounding variable is $z(k) \in Z \subset R^l$, the control variable is $u(k) \in U \subset R^m$, the disturbing variable is $w(k) \in W \subset R^m$, the output variable is $y(k) \in Y \subset R^p$, R is a real number, k, l, m, n and p are integers, $p \le m$; $X^0, Z, U, W$ are respectively open sets of space $R^n, R^l, R^m, R^r$, Y is an open neighborhood in $R^m$, mappings $f: X \times Y \times Z \times U \times W \to R^s$, $s \le n.g : X \times Z \times U \to R^q, q \le n.h: X \to R^p$ are smooth, i.e., arbitrary order derivatives of function f, g and h exist and are continuous,

To solve the system output $\Sigma_1$, the following calculation is performed:

A) For the formula (1), $f(x(k+1), x(k), z(k), u(k), w(k))$ is represented by $x(k+1) = f(x(k), z(k), u(k), w(k))$. In this respect, Equation (1) and (2) are calculated as follows. The first step defines:

$$\rho = rank \frac{\partial}{\partial(\sigma x, z)} F^1(\sigma x, x, z, u, w) =$$

$$rank \frac{\partial}{\partial(\sigma x, z)} \begin{bmatrix} f(\sigma x, x, z, u, w) \\ g(x, u, z) \end{bmatrix}_{(x_e, u_e, w, z_e)}$$

where $F^1 = (f^T, g^T)^T, \sigma^\alpha x(k) = x(k+\alpha)$, $\sigma$ is differential operator, $\alpha$ is differential order, the equilibrium point of the system $\Sigma_1$ is $(x_e, z_e, u_e, w_e)$, and it satisfies

$$f(x_e, x_e, z_e, u_e, w_e) = 0$$

and

$$g(x_e, z_e, u_e) = 0$$

By the above-mentioned algorithm, the following set of equations is obtained.

$$\begin{cases} \tilde{f}(\sigma x, x, u, w, z) = 0 \\ \tilde{g}(x, u, w, z) = 0 \\ \vdots \\ \tilde{\Phi}^{\alpha-1}(\sigma x, \sigma u, \cdots, \sigma^{\alpha-1} u, \sigma w, \cdots, \sigma^{\alpha-1} w) = 0 \end{cases} \qquad (4)$$

$$W^{\alpha-1}(x(0), u(0), \cdots, u(\alpha-2), w(0), \cdots, w(\alpha-2)) = 0 \qquad (5)$$

The solutions are obtained from Equation (4), i.e., by using $x, u, \cdots, \sigma^{\alpha-1} u, w, \cdots, \sigma^{\alpha-1} w$ and $\sigma x^2 = (\sigma x_{s+1}, \cdots, \sigma x_n)$ to express $\sigma x^1 = (\sigma x_1, \cdots, \sigma x_s)$ and $z$, the following can be obtained as

$$\sigma x^1 = \psi(x, u, \cdots, \sigma^{\alpha-1} u, w, \cdots, \sigma^{\alpha-1} w, \sigma x^2) \qquad (6)$$

$$z = \phi(x, u, \cdots, \sigma^{\alpha-1}u, w, \cdots, \sigma^{\alpha-1}w, \sigma x^2) \qquad (7)$$

B) Choosing proper initial conditions for Equation (4) and (5) and determining $\sigma x^2 = \sigma x^{20}$ makes the solution of Equations unique; then choose a dynamic compensator:

$$
\begin{aligned}
T_1(k) &= u(k) \\
T_2(k) &= u(k+1) \\
&\vdots \\
T_{\alpha-1}(k) &= u(k+\alpha-1) \\
u(k+\alpha) &= v(k)
\end{aligned}
$$

The system is divided into

$$x(k+1) = f(x(k), T_1(k), T_2(k), \cdots, T_{\alpha-1}(k), w, \cdots, \sigma^{\alpha-1}w, v(k))$$

i.e., the abbreviation is

$$x(k+1) = f(\overline{x}, v(k), \overline{w})$$

C) Therefore, the original system $\Sigma_1$ can be written as the following expression:

$$\begin{cases} x(k+1) = f(x(k), u(k), w(k)) \\ y(k) = h(x(k)) \end{cases} \qquad (8)$$

By the above operation, the discrete-time singular system has been transformed into the system $\Sigma$ expressed in the following form as

$$
\begin{aligned}
x(t+1) &= f(x(x), u(t), w(t)).x(0) = x_0 \\
y(t) &= h(x(t))
\end{aligned} \qquad (9)
$$

where the state variable is $x(t) \in X^0 \subset R^n$, the control variable is $u(t) \in U \subset R^m$, the disturbing variable is $w(t) \in W \subset R^r$, the output variable is $y(t) \in Y \subset R^p$; $X^0, U, W$ are respectively open sets of space $R^n, R^m, R^r$; $Y$ is an open neighborhood in $R^p$, and mappings $f: X \times U \times W \to X, h: X \to Y$ are analytical.

D) An immersion is defined in the mapping $f(*)$, which is

$$rank_k \frac{\partial f}{\partial(x, u)} = n$$

The following vector space can be defined in the above-mentioned domain $k$.

$$\varepsilon = span_k\{d\varphi, \varphi \in k\}$$

This space $\varepsilon$ can be divided into direct sum of three subspaces, i.e.,

$$\varepsilon = X \oplus U \oplus W$$

where

$$
\begin{aligned}
X &= span_k\{dx(0)\} \\
U &= span_k\{du(k), k \geq 0\} \\
w &= span_k\{dw(k), k \geq 0\}
\end{aligned}
\qquad (10)
$$

define different output space

$$y = span_k\{dy(k), k \geq 0\} \qquad (11)$$

E) By using the inversion algorithm again for the system $\Sigma$, choose the feedback control as follows:

$$u^1(t) = \varphi(x(t), \tilde{v}_i(t+r), v^2(t))$$

$$u^2(t) = v^2(t)$$

$u^1(t)$ and $u^2(t)$ is the output of the system $\Sigma$, which means the system can achieve the state set by the user by controlling this variable.

To solve the $u^1(t)$ and $u^2(t)$, the following inversion algorithm is used again. The following inversion iterating is used for the system $\Sigma$, and denotes

$$d\hat{y}(0) = dy(0) = \frac{\partial h}{\partial x(0)} dx(0) = \bar{a}_0(x(0))dx(0)$$

First step: calculate

$$
\begin{aligned}
d\hat{y}(1) &= \delta\,\bar{a}_0(x(0))dx(1) = \\
&\bar{a}_0(f(x(0), u(0), w(0))[\frac{\partial f}{\partial x(0)} dx(0) + \frac{\partial f}{\partial u(0)} du(0) + \frac{\partial f}{\partial w(0)} dw(0)]) \\
&= a_1 dx(0) + b_1 du(0) + c_1 dw(0)
\end{aligned}
$$

where $a_1, b_1, c_1 \in k$, define $\rho_1 = rank_k b_1$, rearrange components of $y(t)$ to make the row $\rho_1$ of $b_1$ linearly independent, $y(1)$ is decomposed into

$$
\begin{aligned}
d\tilde{y}_1(1) &= \tilde{a}_1 dx(0) + \tilde{b}_1 du(0) + \tilde{c}_1 dw(0) \\
d\hat{y}_1(1) &= \hat{a}_1 dx(0) + \hat{b}_1 du(0) + \hat{c}_1 dw(0)
\end{aligned}
$$

where $\tilde{y}_1(1)$ has $\rho_1$ elements. Because the rows of $\hat{b}_1$ are related to the rows of $\tilde{b}_1$, the matrix exists in $k$ wherein

$M_1(x(0),u(0),w(0))$ satisfies $\hat{b}=M_1\hat{b}_1$, and

$$d\hat{y}_1(1) = \hat{a}_1 dx(0) + M_1[d\tilde{y}_1(1) - \tilde{a}_1 du(0) - \tilde{c}_1 dw(0)] + \hat{c}_1 dw(0)$$
$$= \overline{a}_1 dx(0) + \overline{c}_1 dw(0) + \overline{e}_1^{11} d\tilde{y}_1(1)$$

The (K+1) ($k \geq 0$) step:

Suppose that the k$^{th}$ step is finished and the operation of the first step, $\tilde{y}_1(1), \tilde{y}_2(2),\cdots,\tilde{y}_k(k),\hat{y}_k(k)$ has the following definition,

$$d\tilde{y}_1(1) = \tilde{a}_1 dx(0) + \tilde{b}_1 du(0) + \tilde{c}_1 dw(0)$$
$$\vdots$$
$$d\tilde{y}_k(k) = \tilde{a}_k dx(0) + \tilde{b}_k du(0) + \sum_{j=0}^{k-1}\tilde{c}_k^j dw(j) + \sum_{i=1}^{k-1}\sum_{j=k+1}^{k}\tilde{e}_k^{ij} d\tilde{y}_i(j)$$
$$d\hat{y}_k(k) = \overline{a}_k dx(0) + \sum_{j=0}^{k-1}\tilde{c}_k^j dw(j) + \sum_{i=1}^{k-1}\sum_{j=k+1}^{k}\tilde{e}_k^{ij} d\tilde{y}_i(j)$$

Defining $B_k = (\tilde{b}_1^T,\cdots,\tilde{b}_k^T)^T$ and letting $\rho_k = rank_k B_k$, calculate

$$d\hat{y}_k(k+1) = (\delta\,\overline{a}_k)dx(1) + \sum_{j=0}^{k-1}\delta\,\overline{c}_k^j dw(j+1) + \sum_{i=1}^{k}\sum_{j=i}^{k}\delta\,e_k^{ij} d\tilde{y}_i(j+1)$$
$$= a_{k+1}dx(0) + b_{k+1}du(0) + \sum_{j=0}^{k}c_{k+1}^j dw(j) + \sum_{i=1}^{k}\sum_{j=i}^{k}e_{k+1}^{ij} d\tilde{y}_i(j+1)$$

and define $\rho_{k+1} = rank_k[B_k^T,b_{k+1}^k]^T$, rearrange components of $\hat{y}_k(k+1)$ to make the row $\rho_{k+1}$ of $[B_k^T,b_{k+1}^k]^T$ linearly independent. $\hat{y}_k(k+1)$ is decomposed to

$$d\tilde{y}_{k+1}(k+1) = \tilde{a}_{k+1}dx(0) + \tilde{b}_{k+1}du(0) + \sum_{j=0}^{k}\tilde{c}_{k+1}^j dw(j) + \sum_{i=1}^{k}\sum_{j=i+1}^{k+1}\tilde{e}_{k+1}^{ij} d\tilde{y}_i(j)$$

$$d\hat{y}_{k+1}(k+1) = \hat{a}_{k+1}dx(0) + \hat{b}_{k+1}du(0) + \sum_{j=0}^{k}\hat{c}_{k+1}^j dw(j) + \sum_{i=1}^{k}\sum_{j=i+1}^{k+1}\hat{e}_{k+1}^{ij} d\tilde{y}_i(j)$$

where $\tilde{y}_{k+1}(k+1)$ has $\rho_{k+1}$-$\rho_k$ elements, which are denoted by $B_{k+1} = [B_k^T,\tilde{b}_{k+1}^T]^T$. Because the rows of $\hat{b}_{k+1}$ are related to the rows of $B_{k+1}$, the matrix $M_{k+1}$ satisfying $\hat{b}_{k+1} = M_{k+1}B_{k+1}$ exists in $k$, which makes

$$d\hat{y}_{k+1}(k+1) = \overline{a}_{k+1}dx(0) + \sum_{j=0}^{k}\overline{c}_{k+1}^j dw(j) + \sum_{i=1}^{k+1}\sum_{j=i}^{k+1}\overline{e}_k^{ij} d\tilde{y}_i(j)$$

The (K+1) step finishes.
Let the rank of system $\rho^* = \max\{\rho_k, k \geq 1\}$, and let $\alpha$ be the minimum $k \in n$ satisfying $\rho_k = \rho^*, \alpha \leq n$.
Then the nonlinear system is general immersion, and the regular dynamic measurable disturbance decoupling problem of the system $\Sigma$ expressed in Equation (9) are solvable almost everywhere, if and only if $k \geq 0$,

$$d\hat{y}_k^u(k+1) = \overline{a}_k dx(0) + \sum_{i=1}^{k}\sum_{j=i}^{k}\overline{e}_k^{ij} d\hat{y}_i^u(j)$$

The state feedback $u$ of the above-mentioned system $\Sigma$ is written in the following equations described by the state space $x$ and the new control $v$ :

$$u(t) = \varphi(x(t), v(t), v(t+1), \cdots, v(t+\beta))$$
$$v(t) = \xi(x(t), u(t), u(t+1), \cdots, u(t+\beta)) \tag{12}$$

The one selected is static feedback, because $u$ dose not include the dynamic condition ($\beta = 0$).

According to the above-mentioned algorithm, relative to the $\alpha$ step (also the last step) derived by controlling $u$,

$$d\tilde{y}_1^u(1) = \tilde{a}_1 dx(0) + \tilde{b}_1 du(0)$$
$$\vdots$$
$$d\tilde{y}_\alpha^u(\alpha) = \tilde{a}_{1\backslash\alpha} dx(0) + \tilde{b}_\alpha du(0)$$
$$+ \sum_{i=1}^{\alpha-1} \sum_{j=i+1}^{\alpha} \tilde{e}_\alpha^{ij} dy_i^u(j)$$

the only solution for $du^1 = (du_1,..,du_{\rho*})^T$ is obtained by rearrangement of input, and $du^2 = (du_{\rho*+1},...,du_m)^T$ is defined.

That is to say, the function $\varphi$ and $A_a$ exists almost everywhere, which satisfies local $u^1(t) = \varphi(x(t), \tilde{y}_i^u(t+j), u^2(t))$ where $1 \leq i \leq \alpha$, $i \leq j \leq \alpha$, and

$$\begin{bmatrix} \tilde{y}_1^u(t+1) \\ \vdots \\ \tilde{y}_\alpha^u(t+\alpha) \end{bmatrix} = A_a[x(t), \varphi(*), u^2(t), \tilde{y}_k^u(t+l)]$$

where $1 \leq k \leq \alpha-1$, $k+1 \leq l \leq \alpha$.

Use the following quasi-static feedback

$$u^1(t) = \varphi(x(t), \tilde{v}_i(t+r), v^2(t))$$
$$u^2(t) = v^2(t) \tag{13}$$

where $1 \leq i \leq \alpha$, $0 \leq r \leq \alpha-i$.

F) The Equation (13) is applied to $\Sigma$. The following equations are derived for $k=1,...,\alpha$ :

$$\tilde{y}_k^u(t+k) = \tilde{v}_k(t), t \geq 0 \tag{14}$$

Furthermore, for $k \geq 0$,

$$d\tilde{y}_k^u(k) - \sum_{i=1}^{k} \sum_{j=i}^{k} \bar{e}_k^{ij} d\tilde{y}_i(j) \in X \tag{15}$$

Therefore, the solution of system $\Sigma$ is obtained by the feedback equation (13), so as to obtain the output of system $\Sigma_1$, output variable $\hat{y}_k^u(k)$, which is the required temperature and humidity value.

The instruments used in the control system of the testing environment for formaldehyde and VOC emissions from wood-based panels according to the invention are as follows:

(1) MD604 temperature display and transmitting output instrument: precision of 3%, measuring range and zero point at 0˚ -100˚. The specific drift is corrected when it is used.

(2) MD604 temperature display and transmitting output instrument: precision of 3%, measuring range and zero point at -5˚ -55˚. The specific drift is corrected when it is used.

(3) Humidity transmitting output: according to the change range of the environmental humidity, the humidity transmitter with an appropriate range is chosen, and error is within 1%, and the common high-molecular humidity sensor can meet the requirements.

(4) Temperature transmitting output: according to the change range of the environmental humidity, the temperature transmitter with an appropriate range is chosen, and error is within 1%, and the common thermal resistance and thermocouple sensor can meet the requirements.

In the invention, the constant temperature water tank 6 provides the circulating hot water of a certain temperature to the heat exchanger 18 inside the test chamber 4, so as to heat the test chamber 4 and keep the temperature inside. The refrigerator 12 keeps the water temperature in the dew point water tank 7, so that the humidity generator 16 can supply the test chamber 4 with air which has a definite humidity. By means of the temperature and humidity of the air in the test chamber, according to the above-mentioned dynamic tracing control method, the programmable logic controller PLC can regulate and control the whole system until the temperature and humidity inside the test chamber reach the requirements by detecting the temperature and humidity inside the test chamber.

An example of the specific operation and work process of the dynamic tracing control system is as follows:

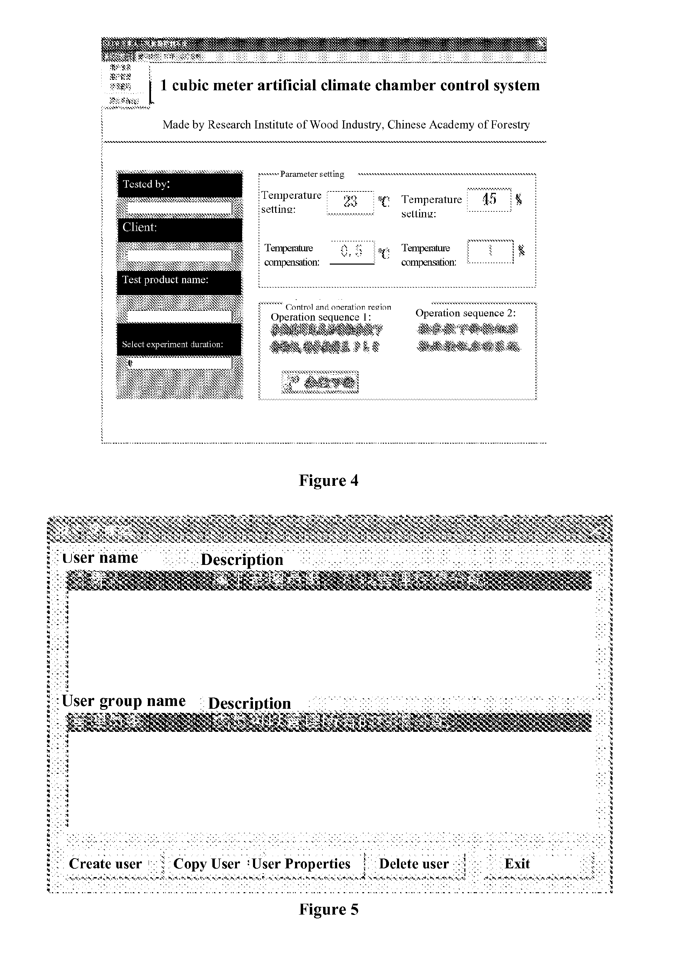

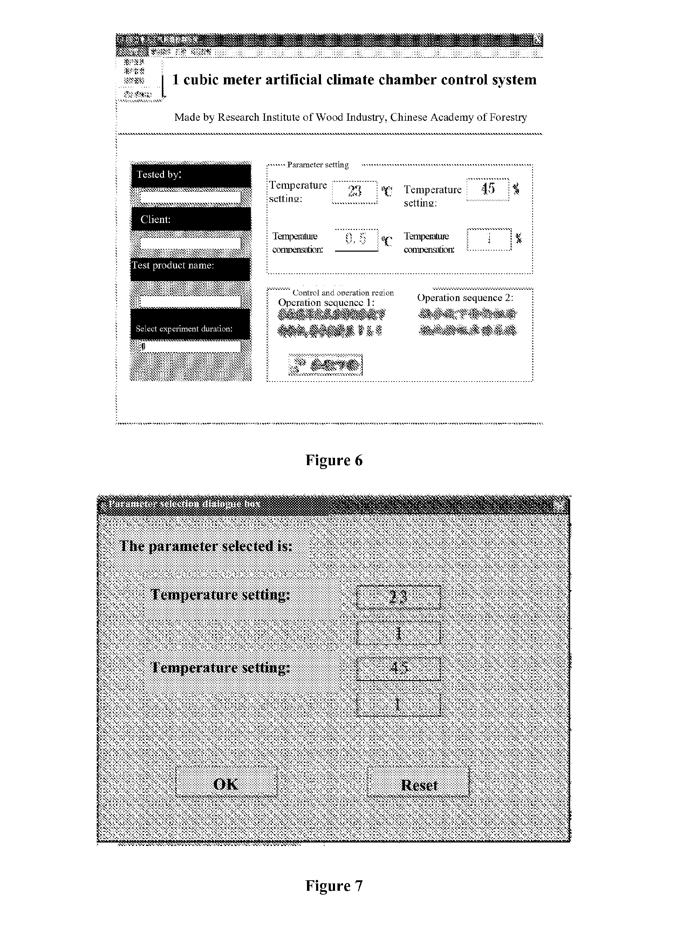

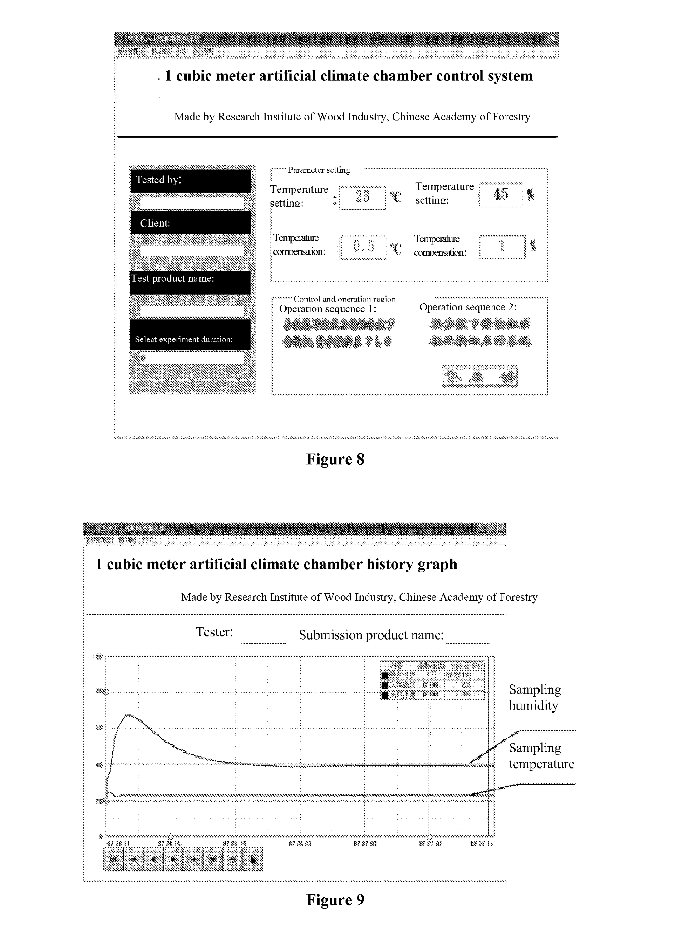

When determining the formaldehyde emissions of some products, the temperature of the test chamber is set as 23˚C, and the humidity is set as 45%. Start the computer, and double click the system icon on the computer desktop. The system first displays the system cover page as shown in Figure 3. Click "OK" (without password when leaving the factory), and after 2 seconds the main interface of the control system appears as shown in Figure 4. Click "System management" from the upper left menu, and a drop-down menu appears. Click "User Management" from the drop-down menu, and the system will show the interface as shown in Figure 5. Add any person in the dialog box who has the authority to use the system. After the adding operation is finished, click "Exit" button, and the system will go back to the main interface as shown in Figure 6. Fill in the Tested by, Client, Tested product name and Date and set the temperature and humidity. When these fields have been filled in, click the "Parameters Download" button, and the system will show a popup dialog box as shown in Figure 7, asking you to confirm that your choice of parameters is correct; re-check the parameters you have set. If they are not correct, click the "Reset" button, and the system will return to the parameters setting interface; if they are correct, click "Enter", and the system will go to the startup interface as shown in Figure 8. Click the "Start" button, and the system starts and enters the real-time monitoring interface (not shown). The device is now operating. According to the PLC's dynamic tracing control, the testing environment is monitored in a real-time manner. In this interface, the operation status of the system can be viewed, and the historical data interface can also be accessed through the window management menu where the user can review the historical data (not shown), or the historical curve interface can be accessed to review the history curve as shown in Figure 9.

After the parameters are downloaded, the system transfers the set parameters to the PLC, which receives the set parameters for tracing control of the system. After 1.5 to 2 hours, the test chamber can reach the test environment requirements as shown in Figure 9. It can be seen from the figure that the dynamic tracing control system for the testing environment of formaldehyde and VOC emissions from wood-based panels according to the present invention can control the testing environment more quickly and accurately than any other system in the background art.

Click the "Running Control" menu after the internal air sampling of the test chamber is finished. Select and click "Stop" from the drop-down menu; the system will stop running. Click "Print" from the menu bar, and select the report or curve that you want to print from the drop-down menu. This concludes that a test run of formaldehyde and VOC emissions from wood-based panels is finished.

Compared with prior temperature and humidity control systems, the present invention has the following obvious advantages:

1. High control precision: the temperature control precision inside the test chamber can reach 0.3%; the humidity control precision can reach 1%, compared to the control precision of 1% for temperature and 3% for humidity of the prior temperature control systems.

2. Rapid response time: the time it takes from machine startup to the point that the testing conditions are met is only 0.5 to 1 hour, as compared to 2 to 4 hours for a prior system to reach the testing conditions.

3. Stable and reliable performance: The general instruments and Siemens' CPU with analog input module are used, and the test chamber can run all year round.

4. Wide range of applications: The adjustable range of the temperature of the present invention temperature is -5˚ to -55˚; the adjustable range of the humidity is 20% to 85%.

5. Use of computer control: The man-machine interface is very friendly, and the operation is simple. The testing data can be represented through tables or curves.

6. Low energy consumption: The power consumption in normal operation of the test chamber of the invention is about 600W.

## Claims

1. A dynamic tracing control system for the testing environment of formaldehyde and VOC emissions from wood-based panels, comprising a computer, a programmable logic controller (PLC), a test chamber (4), a temperature control subsystem (6, 13, 18) and a humidity control subsystem (15, 8, 11, 16, 10, 17, 7, 9); said temperature control subsystem and said humidity control subsystem are connected to said test chamber respectively; said temperature control subsystem (6, 13, 18) is used for heating the testing environment in said test chamber, and said humidity control subsystem (15, 8, 11, 16, 10, 17, 7, 9) supplies the test chamber with air of certain pressure, temperature and humidity; a temperature and humidity sensor (5) is set in said test chamber, and said programmable logic controller is connected to said temperature and humidity sensor, said temperature control subsystem (2, 3) and said humidity control subsystem (4, 10, 7, 8, 11), respectively, and the PLC controls said temperature control subsystem and humidity control subsystem according to the signals collected by said temperature and humidity sensor, so as to control the testing environment inside the test chamber to the required temperature and humidity; **characterized in that** said control program of the PLC is established by taking into consideration such interference factors as external environment, conditions and atmospheric pressure, and the temperature and humidity are master variables represented by $x_n(t)$ ; the uncertainty in the climatic chamber, such as dry bulb temperature, wet bulb temperature and vapor pressure, is regarded as the internal disturbance represented by $Q_n(t)$; the uncertainty of the external environment, such as temperature, humidity and atmospheric pressure, is regarded as the external disturbance represented by $q_n(t)$; $x_n(t)$ is input, $y_n(t)$ is output, and z(t) is feedback; a discretized system dynamics model system $\Sigma_1$ is established for the testing environment in test chamber according to the settings stated in the above, which is represented by:

$$x(k+1) = f(x(k), z(k), u(k), w(k))$$

$$g(x(k), z(k), u(k)) = 0$$

$$y(k) = h(x(k))$$

where the state variable is $x(k) \in X^o \subset R^n$, the bounding variable is $z(k) \in Z \subset R^l$, the control variable is $u(k) \in U \subset R^m$, the disturbing variable is $w(k) \in W \subset R^m$, the output variable is $y(k) \in Y \subset R^p$, R is a real number, k, l, m, n and p are integers, $p \leq m$; $X^0$, Z, U, W are respectively open sets of space $R^n, R^l, R^m, R^r$, Y is an open neighborhood in $R^m$, mappings $f : X \times Y \times Z \times U \times W \rightarrow R^s$, $s \leq n.g : X \times Z \times U \rightarrow R^q, q \leq n.h : X \rightarrow R^p$ are smooth, i.e., arbitrary order derivatives of function f, g and h exist and are continuous;
the following calculations are executed for said system $\Sigma_1$ :

A) define

$$\rho = rank \frac{\partial}{\partial(\sigma x, z)} F^1(\sigma x, x, z, u, w) =$$

$$rank \frac{\partial}{\partial(\sigma x, z)} \left[ \begin{array}{c} f(\sigma x, x, z, u, w) \\ g(x, u, z) \end{array} \right]_{(x_e, u_e, w, z_e)}$$

where $F^1 = (f^T, g^T)^T, \sigma^\alpha x(k) = x(k+\alpha)$, σ is differential operator, α is differential order, the equilibrium point of system $\Sigma_1$ is $(x_e, z_e, u_e, w_e)$, and it satisfies

$$f(x_e, \dot{x}_e, z_e, u_e, w_e) = 0$$

and

$$g(x_e, z_e, u_e) = 0 \; ;$$

B) choose a dynamic compensator

$$
\begin{aligned}
T_1(k) &= u(k) \\
T_2(k) &= u(k+1) \\
&\vdots \\
T_{\alpha-1}(k) &= u(k+\alpha-1) \\
u(k+\alpha) &= v(k)
\end{aligned}
$$

C) by using the calculation mentioned above, the discrete-time singular system $\Sigma_1$ is transformed into the following form:

$$x(t+1) = f(x(x), u(t), w(t)).x(0) = x_0$$

$$y(t) = h(x(t))$$

where the state variable is $x(t) \in X^0 \subset R^n$, the control variable is $u(t) \in U \subset R^m$, the disturbing variable is $w(t) \in W \subset R^r$, the output is $y(t) \in y \subset R^p$ ; t is the time variable,
$X^0, U, W$ are respectively open sets of space $R^n, R^m, R^r$ ; Y is an open neighborhood in $R^p$, mappings $f : X \times U \times W \to X, h : X \to Y$ are analytical;
D) the mapping $f(*)$ defines an immersion, i.e.,

$$rank_k \frac{\partial f}{\partial(x,u)} = n$$

E) by using the inversion algorithm again for the system $\Sigma$, choose the feedback control as follows:

$$u^1(t) = \varphi(x(t), \tilde{v}_i(t+r), v^2(t))$$

$$u^2(t) = v^2(t)$$

where $1 \le i \le \alpha$, $0 \le r \le \alpha\text{-}i$;
$u^1(t)$ and $u^2(t)$ is the output of the system $\Sigma$, which means the system can achieve the state set by the user by controlling this variable;
F) by using the inversion algorithm again, the following expression is derived for $k = 1, \cdots, \alpha$ :

$$\tilde{y}_k^u(t+k) = \tilde{v}_k(t), t \ge 0$$

and for $k \ge 0$, the expression is

$$d\hat{y}_k^u(k) - \sum_{i=1}^{k}\sum_{j=i}^{k} \overline{e}_k^{ij} d\tilde{y}_i(j) \in X$$

thereby the temperature and humidity required by the system $\Sigma_1$, i.e. the output variable $\hat{y}_k^u(k)$, are obtained.

**2.** The dynamic tracing control system for the testing environment of formaldehyde and VOC emissions from wood-based panels according to claim 1, **characterized in that** said temperature control subsystem (6, 13, 18) further comprises a constant temperature water tank (6), a water pump (13) and a heat exchanger (18) which is set in the test chamber (4); said programmable logic controller PLC controls the heaters in the constant temperature water tank according to the dynamic tracing control program, thereby controlling the temperature of the water tank, and controls the water supply of the heat exchanger by controlling the water pump operation.

**3.** The dynamic tracing control system for the testing environment of formaldehyde and VOC emissions from wood-based panels according to claim 2, **characterized in that** said humidity control subsystem (15, 8, 11, 16, 10, 17, 7, 9) further comprises an air filter (15), an air regulating valve (8), an air pump (11), a humidity generator (16), a humidity regulating valve (10) and a humidifier (17), which are connected with moisture pipes; besides, said humidity control subsystem also comprises a dew point water tank (7) and a magnetic pump (9), which are connected with water pipes; said test chamber (4) comprises a humidifier (17) connected to said humidity regulating valve (10) through moisture pipes, a heat exchanger (18) connected to said water pump (13) through hot water pipes and fans (14) connected to the PLC; by the control bus, the PLC controls the constant temperature water tank, dew point water tank, air regulating valve, magnetic pump, humidity regulating valve, air pump, refrigerator, water pump and fans.

**4.** The dynamic tracing control system for the testing environment of formaldehyde and VOC emissions from wood-based panels according to claim 3, **characterized in that** said dynamic tracing control system further comprises a temperature display and transmitting output instrument (19) and a humidity display and transmitting output instrument (20), which are used for displaying the sampling values of temperature and humidity in the climatic chamber; said computer is used to input and display the parameters set by the user, the test-related information and test results.

**Figure 1**

$$q_1(t) \quad q_2(t) \cdots q_n(t)$$

Adjustment

$e(t)$

$p(t)$

$x_n(t)$

$-$

$W_0(s)$

$y_n(t)$

$$Q_1(t) \quad Q_2(t) \cdots Q_n(t)$$

$z(t)$

Transmitting measurement

**Figure 2**

**User login**

User name: Administrator

Password:

Is a member of the administrator group which can manage the authorized assignment

| 1 | 2 | 3 | 4 | 5 | 6 | 7 | 8 | 9 | 0 | ← |
| A | B | C | D | E | F | G | H | I | J | Del |
| K | L | M | N | O | P | Q | R | S | T | Cap |
| U | V | W | X | Y | Z | OK | | | Cancel | |

**Figure 3**

**1 cubic meter artificial climate chamber control system**

Made by Research Institute of Wood Industry, Chinese Academy of Forestry

Tested by:

Client:

Test product name:

Select experiment duration:

Parameter setting

Temperature setting: 23 °C

Temperature setting: 45 %

Temperature compensation: 0.5 °C

Temperature compensation: 1 %

Control and operation region

Operation sequence 1:

Operation sequence 2:

**Figure 4**

User name    Description

User group name    Description

Create user | Copy User | User Properties | Delete user | Exit

**Figure 5**

Figure 6

Figure 7

**. 1 cubic meter artificial climate chamber control system**

Made by Research Institute of Wood Industry, Chinese Academy of Forestry

Tested by:

Client:

Test product name:

Select experiment duration:

Parameter setting

Temperature setting: 23 °C    Temperature setting: 45 %

Temperature compensation: 0.5 °C    Temperature compensation: 1 %

Control and operation region
Operation sequence 1:    Operation sequence 2:

**Figure 8**

**1 cubic meter artificial climate chamber history graph**

Made by Research Institute of Wood Industry, Chinese Academy of Forestry

Tester:    Submission product name:

Sampling humidity

Sampling temperature

**Figure 9**

# INTERNATIONAL SEARCH REPORT

| | International application No. |
|---|---|
| | PCT/CN2008/070193 |

**A. CLASSIFICATION OF SUBJECT MATTER**

see extra sheet

According to International Patent Classification (IPC) or to both national classification and IPC

**B. FIELDS SEARCHED**

Minimum documentation searched (classification system followed by classification symbols)

IPC: G05D27, G05D22, G05D23, G01N33

Documentation searched other than minimum documentation to the extent that such documents are included in the fields searched

Electronic data base consulted during the international search (name of data base and, where practicable, search terms used)

WPI&EPODOC&PAJ    climat+, environment, temperature, humidity

**C. DOCUMENTS CONSIDERED TO BE RELEVANT**

| Category* | Citation of document, with indication, where appropriate, of the relevant passages | Relevant to claim No. |
|---|---|---|
| A | CN101025424A(ZHOU Yucheng) 29 Aug.2007(29.08.2007) the whole document | 1-4 |
| A | ZHOU Yucheng et al. Dynamic measurable disturbance decoupling for discrete time singular nonlinear systems.Control and Decision. July 2004, vol.19, No.7, pages 787-790 | 1-4 |
| A | ZHOU Yucheng et al. Exact Linearization of a Class of Implicit Discrete-time Nonlinear Singular Systems. Control and Decision. April 1998, vol.13, No.4, pages317-321 | 1-4 |
| A | CN1439943A (ZHOU Yucheng) 03 Sep.2003(03.09.2003) the whole document | 1-4 |
| A | US2003/0172751A1(MALMYR L et al)18 Sep.2003(18.09.2003) the whole document | 1-4 |

☒ Further documents are listed in the continuation of Box C.     ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&"document member of the same patent family |

| Date of the actual completion of the international search | Date of mailing of the international search report |
|---|---|
| 06 Oct.2008(06.10.2008) | **06 Nov. 2008 (06.11.2008)** |
| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br><br>SUN, Chunmei<br><br>Telephone No. (86-10)62085674 |

Form PCT/ISA/210 (second sheet) (April 2007)

**EP 2 287 701 A1**

## INTERNATIONAL SEARCH REPORT

International application No.

PCT/CN2008/070193

C (Continuation). DOCUMENTS CONSIDERED TO BE RELEVANT

| Category* | Citation of document, with indication, where appropriate, of the relevant passages | Relevant to claim No. |
|---|---|---|
| A | CN2656984Y(ZHOU Yucheng)17 Nov.2004(17.11.2004) the whole document | 1-4 |

Form PCT/ISA/210 (continuation of second sheet ) (April 2007)

22

<div style="text-align:center">

**INTERNATIONAL SEARCH REPORT**
Information on patent family members

</div>

International application No.

PCT/CN2008/070193

| Patent Documents referred in the Report | Publication Date | Patent Family | Publication Date |
|---|---|---|---|
| CN101025424A | 29 Aug.2007 | None | |
| CN1439943A | 03 Sep.2003 | CN1211718C | 20 Jul.2005 |
| US2003/0172751A1 | 18 Sep.2003 | SE9903327A | 17 Mar.2001 |
| | | WO0119519A | 22 Mar.2001 |
| | | AU7464000A | 17 Apr.2001 |
| | | SE515139C | 18 Jun.2001 |
| | | EP1272275A | 08 Jan.2003 |
| | | JP2003509664T | 11 Mar.2003 |
| | | AT254505T | 15 Dec.2003 |
| | | DE60006726T | 30 Sep.2004 |
| | | US2005145047A | 07 Jul.2005 |
| CN2656984Y | 17 Nov.2004 | None | |

Form PCT/ISA/210 (patent family annex) (April 2007)

23

| INTERNATIONAL SEARCH REPORT | International application No. |
|---|---|
| | PCT/CN2008/070193 |

According to International Patent Classification (IPC) or to both national classification and IPC:

G05D27/00(2006.01)I

G05D22/00(2006.01)I

G05D23/00(2006.01)I

G01N33/46(2006.01)I

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• *World Health Organization's,* 1990